# EUROPÄISCHE PATENTSCHRIFT

(11) **EP 1 641 442 B1**
(45) Veröffentlichungstag und Bekanntmachung des Hinweises auf die Patenterteilung: **15.09.2010**
(21) Anmeldenummer: 04740552.7
(22) Anmeldetag: 02.07.2004
(51) Int. Cl.: A61K 31/505, A61P 11/00

(54) **VERWENDUNG VON ECTOIN UND/ODER HYDROXYECTOIN ZUR HERSTELLUNG VON INHALIERBAREN ARZNEIMITTELN SOWIE EINE DIES ENTHALTENDE INHALATIONSVORRICHTUNG**
USE OF ECTOINE AND/OR HYDROXYECTOINE FOR THE PRODUCTION OF INHALABLE MEDICAMENTS AND AN INHALATION DEVICE COMPRISING THIS AGENT
UTILISATION D'ECTOINE ET/OU HYDROXYECTOINE POUR LA PRODUCTION DE MEDICAMENTS INHALABLES ET DISPOSITIF D'INHALATION CONTENANT CET COMPOSANT

(30) Priorität: 07.07.2003 DE 10330768
(43) Veröffentlichungstag der Anmeldung: 05.04.2006
(73) Patentinhaber: Bitop Aktiengesellschaft Für Biotechnische Optimierung, 58453 Witten (DE)
(72) Erfinder: KRUTMANN, Jean, 41844 Wegberg (DE)
(74) Vertreter: Schneiders, Josef
(86) Internationale Anmeldenummer: PCT/EP2004/007189
(87) Internationale Veröffentlichungsnummer: WO 2005/002556

(56) Entgegenhaltungen:
- WO-A-01/76572
- DE-A- 10 006 578

## Beschreibung

Die menschliche Gesundheit wird zunehmend durch Umweltnoxen beeinträchtigt. Besonders zu nennen ist hier die Luftverschmutzung mit Schwebstäuben, die faserförmiger oder partikulärer Natur sein können. In epidemiologischen Untersuchungen wurde gezeigt, dass Schwebstäube an der Entstehung von Lungenerkrankungen und kardiovaskulären Erkrankungen beteiligt sind. In großen europäischen Städten sind jährlich 60.000 Todesfälle die Folge einer langanhaltenden Luftverschmutzung. An dieser Luftverschmutzung sind Schwebstäube maßgeblich beteiligt. Es dürfte in absehbarer Zeit de facto unmöglich sein, die Belastung mit Schwebstäuben, insbesondere mit feinen und ultrafeinen Schwebstäuben durch Fittermassnahmen signifikant zu reduzieren. Es ist vielmehr mit einem erheblichen Ansteigen dieser Belastungen zu rechnen. Aus der URL *http:*//*propu*/*mone.ch*/*Staubpartike*/ ist es bekannt dass der Mensch im Laufe seines Lebens mindestems 400.000 m³ Luft einatmet. Bei einer mittleren Partikelkonzentration in der Aussenluft von 30 µg/m³ und einem in der Lunge zurückgehaltenen Anteil von angenommen nur 20% werden pro Tag in jedem der 300 Millionen Alveolen etwa 100 Partikel abgelagert. In Raucherhaushalten ist die Belastung etwa 20 bis 45 % höher anzunehmen. Die gesundheitsschädlichen Wirkungen der Schwebstäube beruhen auf einer Interaktion dieser Moleküle mit dem menschlichen Lungengewebe. Die Folge davon sind entzündliche, zuweilen sogar maligne Lungenerkrankungen. In die Lunge gelangte staubförmige Fremdstoffe sind somit eine der wesentlichen Ursachen für die Entstehung von Lungenkrankheiten.

Außerdem wird angenommen, dass die Aufnahme von Schwebstäuben über die Lungenzellen und die in diesen Zellen in Folge ausgelösten biologischen Wirkungen auch für die Pathogenese kardiovaskulärer Erkrankungen zumindest mitverantwortlich sind.

Daraus folgt, dass die in der Industriegesellschaft immer mehr zunehmende Belastung der Lunge durch Schwebstäube für eine nicht unbeträchtliche Erhöhung krankheitsbedingter Todesfälle verantwortlich gemacht werden muß.

Aus obiger URL ist außerdem bekannt geworden, dass nach neueren Studien gesundheitliche Folgen sogar bei bisher als unbedenklich eingestuften Konzentrationen von Schwebstäuben anzunehmen sind. In verschiedenen Großstädten wurden Untersuchungen durchgeführt, die ergaben, dass mit der Erhöhung des Tageswertes der Staubbelastung um nur 10 µg/m³ eine Zunahme nicht unfallbedingter Todesfälle um 0,5 bis 1 % einhergeht. Da, wie gesagt, die Konzentration an Schwebstaub in der Luft nicht wirklich gesenkt werden kann und der Gebrauch von Atemluft-Filtern auf Ausnahmefälle beschränkt bleiben muss, muss nach allgemein und einfach anwendbaren Mitteln gesucht werden, mit denen die Belastung der menschlichen Lunge vermindert und so die bekannten Folgeschäden möglichst gering gehalten werden können.

Es ist vor allem dringend geboten, für Menschen, die der täglichen Belastung mit Schwebstäuben z.B. am Arbeitsplatz nicht aus dem Weg gehen können, insbesondere für diejenigen, die an einer besondere Empfindlichkeit gegenüber Schwebstäuben leiden, vorbeugende Maßnahmen, zu entwickeln, die allgemein, einfach und jederzeit eingesetzt werden können.

Aufgabe der Erfindung ist es daher, pharmazeutische Mittel bereitzustellen, die im Stande sind, die oben beschriebenen negativen Auswirkungen von Schwebstäuben auf die Gesundheit des Menschen, insbesondere pulmonale und kardiovaskuläre Erkrankungen, wirksam zu bekämpfen

Es wurde gefunden, dass Arzneimittel in inhalierbarer Form, welche als Wirkstoff Ectoin, Hydroxyectoin und/oder deren Salze enthalten, überraschend eine wirksame Prophylaxe gegen die oben beschriebenen Krankheitszustände und deren Behandlung ermöglichen.

Ectoine werden aus extremophilen Bakterien gewonnen. Dies sind außergewöhnliche Mikroorganismen, die fähig sind, unter extremen Bedingungen, z.B. bei biologisch extremen Salzkonzentrationen bis 200 g Kochsalz pro Liter und bei Temperaturen im Bereich von 60 bis 110°C zu leben und sich zu vermehren. Solche Lebensbedingungen würden bei normalen (mesophilen) Organismen zum sofortigen Tod und mindestens zu massiven Schädigungen zellulärer Strukturen führen.

In den letzten Jahren wurde daher ein großer Forschungsaufwand betrieben, um diejenigen biochemischen Komponenten zu identifizieren, auf welche die bemerkenswerte thermische, chemische und physikalische Stabilität der Zellstrukturen extremophiler Organismen zurückzuführen ist.

Zu der hohen Temperaturstabilität von Zellstrukturen tragen in erheblichem Maß niedermolekulare organische Substanzen im intrazellulären Milieu bei, die als Osmolyte oder kompatible Solute bezeichnet werden. Die in den extremophilen Mikroorganismen gefundenen Osmolyte werden von menschlichen oder tierischen Zellen nicht gebildet.

In neuerer Zeit konnten in extremophilen Mikroorganismen erstmals verschiedene neuere Osmolyte identifiziert werden. Hierzu gehören beispielsweise Ectoin, Hydroxyectoin, Firoin, Firoin-A, Diglycerolphosphat, cyclisches Diphosphoglycerat, Diinositolphosphat und 1,3-Di-mannosyl-di-myoinositoiphosphat (DMIP).

Alle werden aus extremophilen Mikroorganismen gewonnen und aufgearbeitet bzw. gereinigt (vgl. EP-A 94 903 874; EP-A 98 121 243; DE-A 100 47 444) und bilden eine bekannte Gruppe niedermolekularer Stoffe mit Schutzeigenschaften für ansonsten sensible Zellen. In einigen Fällen konnte der Beitrag dieser Verbindungen zum Schutz von Hautzellen gegenüber externen Stressbedingungen wie Hitze- und Trokkenheit auf dem Gebiet der Kosmetik ( vgl. US-A 6 267 973, ) gezeigt werden. Verschiedentlich wurde auch schon vorgeschlagen, topische Arzneimittel zum Schutz der Haut vor externen Stresseinwirkungen oder zur Behandlung von Erkrankungen einzusetzen, die durch den enzymatischen Abbau von Gewebestrukturen verursacht werden ( vgl. DE-A 100 06 578). Aufgeführt wurden neben anderen Krankheiten allgemein Erkrankungen des Immunsystems, Autoimmunerkrankungen, Entzündungsprozesse sowie akute und chronische Entzündungen.

Die ebenfalls auf die Verwendung von Osmolyten gerichtete DE-A 198 34 816 betrifft Kosmetika mit einer Schutzwirkung gegen die UV-Bestrahlung der Haut, die daneben auch eine Wirksamkeit bei der Stabilisierung von Nukleinsäuren menschlicher Hautzellen aufweisen sollen. Das Osmolyt Ectoin wurde auch als Feuchtigkeitsspender (moisturizer) in kosmetischen Produkten eingesetzt, mit dem Ziel, die menschliche Haut gegen schädigende Wirkungen der ultravioletten Sonnenstrahlung zu schützen (EP-A 19 990 941).

WO 01/76572 beschreibt die orale oder topische Verwendung von Soluten zum Schutz von Organismen vor Erkrankungen z.B. des Herzens, des respiratorischen Trakts, die durch freie Radikale ausgelöst werden.

Die Herstellung eines Arzneimittels zur allgemeinen Behandlung von Hauterkrankungen mittels Osmolyten, insbesondere Ectoin oder Hydroxyectoin ist aus der EP-A 0 887 418 bekannt, die von der Anmelderin Bitop AG selbst hinterlegt wurde. Hierbei ging man davon aus, dass diese Wirkstoffe zur Stabilisierung von Enzymen und anderen Biomolekülen beitragen und daher zur Stabilisierung denaturierender Bedingungen beitragen können.

In den Offenlegungsschriften DE-A 199 33 460, DE-A 199 33 461, DE-A 199 33 463 und DE-A 199 33 466 ist vorgeschlagen worden, Ectoine aufgrund einer antioxidativen Wirkung als Radikalfänger einzusetzen, und damit die Haut insbesondere vor der durch Sonnenbestrahlung beschleunigten und verstärkten Hautalterung zu schützen. Damit sollten auch unerwünschte Zustände der Haut, die aus oxidativen Vorgängen resultieren, vermieden werden. Die WO 01/72287 beschreibt unter ähnlichen Prämissen wie in den zuletzt genannten Druckschriften vorgeschlagen, die Anwendung von Ectoinen bei der Behandlung von UV-induzierter Irnmunsuppression.

Bisher ist nicht bekannt, wie Osmolyte auf anderes Gewebe als das der Haut einwirken. Bereits die äußerliche Applikation von Hydroxyectoin auf Cornea und Iris des Kaninchenauges ( vgl. Heusener-Report No. T14952 v. 06:04.2001) verursachten anfangs Irritationen und Reizungen an der Bindehaut (Rötung, Chemose und Discharge), die zwar später nicht mehr beobachtet wurden, aber dem Fachmann einen klaren Hinweis auf zu erwartende Unverträglichkeiten bei empfindlicheren Gewebeoberflächen geben mußten. Angesichts dieser Erkenntnisse lag es für den Fachmann sicher nicht nahe, Osmolyte als zwar natürliche, aber schon aufgrund ihrer "unnatürlichen" Entstehung als körperfremde Wirkstoffe verdächtige Substanzen mit menschlichem Lungengewebe in Kontakt zu bringen. Eine Anwendung der Osmolyte am Gewebe im Körperinneren, insbesondere am äußerst reizempfindlichen Lungen- oder Bronchialgewebe, wurde daher vom Fachmann bisher offenbar überhaupt nicht in Betracht gezogen.

Überraschenderweise hat sich nun aber gezeigt, dass Ectoin und Hydroxyectoin vom menschlichen Bronchial- und Lungengewebe, einschließlich der Alveolen, nicht nur gut vertragen werden, sondern unerwartet auch eine hervorragende prophylaktische Wirkung gegen die schädlichen Einwirkungen von Schwebstäuben ausüben, unabhängig davon, welcher Natur die Schwebstäube sind. Sie eignen sich auch zur Behandlung von durch diese Einwirkungen kausal bedingten Erkrankungen.

Es ist somit möglich, durch eine geeignete Prophylaxe unq/oder Behandlung mittels entsprechend dosierten Inhalationsformen, welche Ectoin und/oder Hydroxyectoin als Wirkstoff enthalten, nicht nur die allgemein bekannten und oben beschriebenen Lungenerkrankungen, sondern auch die hieraus entstehenden kardiovaskulären Erkrankungen wirksam zu bekämpfen.

Gegenstand der Erfindung ist die Verwendung von Ectoin und/oder Hydroxyectoin, sowie von deren pharmakologisch verträglichen Salzen, zur Herstellung von Arzneizubereitungen in inhalierbarer Form zur Bekämpfung von auf Schwebstaubeinwirkung auf das Lungengewebe beruhenden Krankheiten und/oder hiermit verbundenen kardiovaskulären Erkrankungen.

Ein weiterer Gegenstand der Erfindung ist eine mit Wirkstoff befüllte Inhalationsvorrichtung deren zerstäubbarer fester oder flüssiger Inhalt aus einer mindestens ein Osmolyt oder dessen Derivate und/oder pharmakologisch verträgliche Salze enthaltenden Wirkstoffzusammensetzung besteht.

Unter der Bekämpfung von Lungenkrankheiten wird folglich erfindungsgemäß sowohl die Prophylaxe bei gesunden Menschen, als.auch die Behandlung von Menschen verstanden, bei denen die Symptome der Schwebstaubeinwirkung schon bestehen.

Einige der erfindungsgemäßen Wirkstoffe sind schwache Basen oder Säuren und können daher auch, in manchen Fällen sogar bevorzugt, in ihrer pharmakologisch besonders verträglichen neutralen Salzform zum Einsatz kommen.

Als pharmakologisch verträgliche Salze kommen die Alkali- oder Erdalkalisalze, insbesondere die Salze des Kalium, Natrium, Magnesium und Calcium, aber auch Salze mit organischen Basen wie z.B. mit nicht toxischen aliphatischen oder aromatischen Aminen in Frage.

Überwiegt im Falle der Anwesenheit von Stickstoffatomen im Wirkstoffmolekül die basische Natur, werden Salze mit pharmakologisch unbedenklichen organischen oder anorganischen Säuren, wie z.B. Essigsäure, Citronensäure, Weinsäure, Mandelsäure, Äpfelsäure, Milchsäure, Chlorwasserstoffsäure, Bromwasserstoffsäure, Schwefelsäure oder Phosphorsäure gebildet.

Erfindungsgemäße Wirkstoffe sind Ectoin, Hydroxyectoin sowie deren Salze. Ebenfalls bevorzugt sind Kombinationspräparate, die beide Wirkstoffe nebeneinander ggf. mit weiteren Wirkstoffen enthalten.

Allgemein können die erfindungsgemäßen Wirkstoffe oder deren Kombinationen ggf. auch mit weiteren Wirkstoffen unter Einsatz von für die Inhalationstherapie üblichen und pharmakologisch unbedenklichen Hilfs- und Zusatzstoffen in bekannter Weise zu inhalierbaren Arzneimitteln verarbeitet werden.

Solche Zusätze sind bei inhalierbaren flüssigen Zubereitungen wegen der leichten Wasserlöslichkeit in erster Linie steriles Wasser ggf. unter Zusatz von weiteren Lösungsmitteln, Stabilisatoren, Konservierungsmitteln oder Lösungsvermittlern.

Als Aerosole werden Stoffsysteme bezeichnet, die aus festen und/oder flüssigen Partikeln bestehen, die fein in einem Gas verteilt sind. Im üblichen Sinn werden meist Wirkstoffe enthaltende Flüssigkeiten oft in Form von Lösungen zu Aerosolen versprüht.

Besonders praktisch in der Anwendung von Feststoffgemischen in sogenannten Pulverinhalatoren, mit denen diese Feststoffe für die Inhalation bereitgestellt werden können. Für die Verabreichung der Wirkstoffe stehen verschiedene Gerätetypen zur Verfügung die auf unterschiedlichen Sprühmechanismen beruhen, hierzu gehören z.B. Spinhaler, Diskhaler, Turbohaler, Rotahaler oder Aerolizer.

Erfindungsgemäß ist es grundsätzlich zu empfehlen, den Anteil an Hilfsstoffen im Inhalat auf ein Minimum zu begrenzen und für Pulverinhalatoren nur leicht resorbierbare nichtreizende Trägerstoffe wie z.B. mikronisierte Laktose einzusetzen. Entsprechend mikronisierte Feststoffe sind als Trägersubstanzen besonders brauchbar, welche die Wirkstoffe in adsorbierter oder absorbierter Form enthalten. Diese Form der Inhalationstherapie hat sich in jüngerer Zeit mehr und mehr durchgesetzt, Die neueren Feststoffinhalatoren ermöglichen eine besonders einfache und sichere Applikation.

Es können neben den erfindungsgemäß vorgeschlagenen Wirkstoffen auch weitere für die Behandlung ggf. geeignete Wirkstoffe zugesetzt werden. Hierzu gehören beispielsweise Antiasthmatika, Broncholytika oder Expektorantia.

Flüssige Dosier-Aerosole können mit üblichen Treibmitteln, wie z.B den FCKW-Treibmitteln Dichlor-difluor-methan, Trichlor-fluormethan oder Cryofluoran verwendet werden. Bevorzugt sind halogenfreie Treibmittel wie Propan oder Butan oder komprimierte untoxische Gase wie Stickstoff, Kohlendioxid oder Distickstoffmonoxid.

Die erfindungsgemäßen Wirkstoffe, können praktisch zu allen inhalierbaren Zubereitungsformen verarbeitet werden. Solche Zubereitungsformen sind beispielsweise Lösungen, Flüssig/Fest-Dispersionen, Fest/Fest-Dispersionen, Suspensionen und Emulsionen

Die Konzentration der Wirkstoffe liegt im Bereich von 0,005 bis 20 Gew.% bezogen auf das Gewicht des eingesetzten Trägermaterials. Bevorzugt ist der Bereich von 0,05 bis 2 Gew.%.

Die folgenden Beispiele dienen zur Erläuterung der Erfindung, beschränken diese jedoch in keiner Weise.

### Beispiel 1

Druckgas-Inhalationsmittel:

| Inhaltstoff | Gehalt in Gewichts-% |
|---|---|
| Wasser | 97,0 |
| Ectoin | 0,5 |
| Hydroxyectoin | 0,5 |
| Konservierungsstoff | 0,2 |
| Treibgas Stickstoff | |

### Beispiel 2

Pulver-Inhalatonsmittel:

| Inhaltstoff | Gehalt in Gewichts-% |
|---|---|
| Lactose mikrokristallin | 99,4 |
| Ectoin | 0,3 |
| Hydroxyectoin | 0,3 |

## Patentansprüche

1. Verwendung von Ectoin und/oder Hydroxyectoin, sowie von deren pharmakologisch verträglichen Salzen, zur Herstellung von inhalierbaren Arzneizubereitungen zur Bekämpfung von auf Schwebstaubeinwirkung auf das Lungengewebe beruhenden Krankheiten und/oder hiermit verbundenen kardiovaskulären Erkrankungen.

2. Verwendung nach Anspruch 1, **dadurch gekennzeichnet, dass** der Zubereitung weitere Wirkstoffe zugesetzt werden.

3. Mit Wirkstoff befüllte Inhalationsvorrichtung, deren zerstäubbarer Inhalt aus einer Ectoin und/oder Hydroxyectoin oder deren pharmakologisch verträgliche Salze enthaltenden Wirkstoffzusammensetzung besteht.

4. Inhalationsvorrichtung nach Anspruch 3 in Form eines gefüllten Inhalators für flüssige Arzneimittel.

5. Inhalationsvorrichtung nach Anspruch 3 in Form eines gefüllten Pulverinhalators.

## Claims

1. Use of ectoine and/or hydroxyectoine as well as of its pharmacologically compatible salts for the preparation of an inhalable pharmaceutical formulation for combating diseases caused by the effects of suspended particulate on the tissue of lungs or the cardiovascular diseases that are related thereto.

2. The use according to claims 1, **characterized in that** the formulation comprises further active agents.

3. An inhalation device filled with active agent, the atomizable content thereof consisting of an active agent composition comprising ectoine and/or hydroxyectoine or a pharmacologically compatible salt thereof.

4. The inhalation device according to claim 3 in form of a filled inhaling device for liquid pharmaceuticals.

5. The inhalation device according to claim 3 in form of a filled powder inhalation device.

## Revendications

1. Utilisation d'ectoïne et/ou d'hydroxyectoïne, ainsi que de leurs sels pharmacologiquement acceptables, pour la préparation de compositions médicamenteuses inhalables pour la lutte contre les maladies reposant sur l'action sur le tissu pulmonaire des poussières en suspension et/ou les affections cardiovasculaires qui y sont liées.

2. Utilisation selon la revendication 1, **caractérisée en ce que** d'autre substances actives sont ajoutées à la composition.

3. Dispositif pour inhalation chargé de substance active, dont la teneur pulvérisable consiste en une composition de substances actives contentant de l'ectoïne et/ou de l'hydroxyectoïne ou leurs sels pharmacologiquement acceptables.

4. Dispositif pour inhalation salon la revendication 3, sous forme d'un inhalateur chargé pour médicament liquide.

5. Dispositif pour inhalation selon la revendication 3, sous forme d'un inhalateur de poudre chargé.
